# EUROPEAN PATENT APPLICATION

(11) **EP 3 011 979 A1**
(43) Date of publication of application: **27.04.2016**
(21) Application number: 14813436.4
(22) Date of filing: 04.03.2014
(51) Int. Cl.: A61L 27/00, A61B 17/68, C22C 23/00, C22C 23/02, C22C 23/04, C22C 23/06, C23C 22/08, C25D 11/30

(54) **IMPLANT FOR LIVING ORGANISMS**

(30) Priority: 18.06.2013 JP 2013127507
(71) Applicant: Olympus Corporation, Shibuya-ku Tokyo 151-0072 (JP)
(72) Inventor: TANIGUCHI, Hirofumi, Tokyo 151-0072 (JP)
(74) Representative: Schicker, Silvia
(86) International application number: PCT/JP2014/055448
(87) International publication number: WO 2014/203566

(57) **Abstract**

It is possible to maintain a base material made of magnesium or a magnesium alloy so as to prevent rapid degradation over a predetermined period after implanting. Provided is a biological implant (1) including a ceramic membrane (3) provided on a surface of a base material (2) made of magnesium or a magnesium alloy, wherein the total content of a metal element contained in the ceramic membrane (3), which has a standard electrode potential equal to or greater than -2.35 V and equal to or less than 0 V, is set to be equal to or less than a value at which the base material (2) can keep a desired mechanical strength over a healing period of an implant site in biological tissue.

## Description

### {Technical Field}

The present invention relates to a biological implant.

### {Background Art}

In the related art, there are known biological implants whose biodegradation rate is controlled by adjusting the concentration of impurity elements to be added, in minute amounts, to a base material made of a biodegradable magnesium-based alloy and a coating layer coating a surface of the base material (for example, see Patent Literature 1).

Minor elements to be added in this Patent Literature 1 are selected from zirconium (Zr), molybdenum (Mo), niobium (Nb), tantalum (Ta), titanium (Ti), strontium (Sr), chromium (Cr), manganese (Mn), zinc (Zn), silicon (Si), phosphorus (P), nickel (Ni), and iron (Fe).

### {Citation List}

### {Patent Literature}

{PTL 1} Japanese Unexamined Patent Application, Publication No. 2012-196461

### {Summary of Invention}

### {Technical Problem}

However, because the above-described trace elements are elements whose standard electrode potentials are higher than that of magnesium and lower than that of hydrogen, the elements react with a body fluid when implanted in biological tissue, which causes ionization thereof, thus leaching out more easily. When the trace elements leach out by being ionized, there is a problem in that magnesium or the magnesium alloy in the base material existing in the area surrounding the trace elements is ionized. Specifically, there is a problem in that, in the case in which the base material is formed of magnesium, in addition to leaching caused by a reaction between the base material itself and the body fluid, a reaction with the ionized trace elements also accelerates leaching of magnesium.

The present invention has been conceived in light of the above-described circumstances, and an object thereof is to provide a biological implant with which it is possible to maintain a base material made of magnesium or a magnesium alloy so as to prevent rapid degradation thereof over a predetermined period after implanting.

### {Solution to Problem}

In order to achieve the above-described object, the present invention provides the following solutions.

An aspect of the present invention is a biological implant including a ceramic membrane provided on a surface of a base material made of magnesium or a magnesium alloy, wherein a total content of a metal element contained in the ceramic membrane, which has a standard electrode potential equal to or greater than -2.35 V and equal to or less than 0 V, is set to be equal to or less than a value at which the base material can keep a desired mechanical strength over a healing period of an implant site in biological tissue.

With this aspect, when the biological implant is implanted in the biological tissue, the ceramic membrane provided at the surface of the base material comes into contact with the biological tissue and is degraded by a reaction with the body fluid, thus ionizing the metal elements contained therein. Among metal ions, although those having standard electrode potentials equal to or greater than -2.35 V and equal to or less than 0 V accelerate the degradation of magnesium or the magnesium alloy in the base material, in this aspect, by suppressing the total content thereof sufficiently low, it is possible to keep the mechanical strength of the base material at a desired value during a period until the biological tissue at the implant site is healed. Accordingly, the biological implant can continue to support the affected area until the affected area is healed.

In the above-described aspect, the desired mechanical strength may be 85 % or greater relative to a mechanical strength before implanting.

By doing so, it is possible to ensure a high enough mechanical strength of the biological implant until the affected area is healed, and it is possible to continue to support the affected area.

In the above-described aspect, it is preferable that the total content be equal to or less than 109 ppm.

By doing so, it is possible to maintain the mechanical strength at about 85 % or greater even after 90 days have passed after implanting in the biological tissue.

In the above-described aspect, it is preferable that the total content be equal to or less than 99 ppm.

By doing so, it is possible to maintain the mechanical strength at about 90 % or greater even after 90 days have passed after implanting in the biological tissue.

Furthermore, in the above-described aspect, it is preferable that the total content be equal to or less than 62 ppm.

By doing so, it is possible to maintain the mechanical strength at about 95 % or greater even after 90 days have passed after implanting in the biological tissue.

In the above-described aspect, the ceramic membrane may contain magnesium and oxygen as a first main component and a second main component, respectively.

In addition, in the above-described aspect, the metal element may be at least one type of metal element selected from Nd, Sm, Dy, Gd, Al, V, Zr, Mn, Zn, Cr, Fe, Ni, Sn, and Pb.

In the above-described aspect, the ceramic membrane may be generated in a membrane-generating processing step after molding the base material.

By doing so, a ceramic membrane in which the content of a specific metal element is reduced by the membrane-generating processing performed after molding the base material, except for a membrane formed during the molding processing and a membrane formed by natural oxidation caused by contact with the atmosphere, can be formed as the ceramic membrane.

In the above-described aspect, the membrane-generating processing step may employ a wet process.

By doing so, by controlling the metal-element contents of a reagent in which the base material is immersed in the membrane-generating processing step, it is possible to precisely control the contents of metal elements having standard electrode potentials equal to or greater than -2.35 V and equal to or less than 0 V.

In the above-described aspect, the membrane-generating processing step may employ an anodic oxidation process.

By doing so, it is possible to enhance the adhesion strength between the generated ceramic membrane and the base material, and it is possible to enhance the corrosion resistance by preventing the membrane from physically peeling off.

In the above-described aspect, the membrane-generating processing step may employ a chemical conversion coating.

By doing so, it is possible to use high-purity reagents as the reagents to be used in the chemical conversion coating, such as acid treatment or the like, which facilitates manufacturing management, and it is possible to more stably enhance the corrosion resistance.

### {Advantageous Effects of Invention}

With the present invention, an advantage is afforded in that it is possible to maintain a base material made of magnesium or a magnesium alloy so as to prevent rapid degradation thereof over a predetermined period after implanting.

### {Brief Description of Drawings}

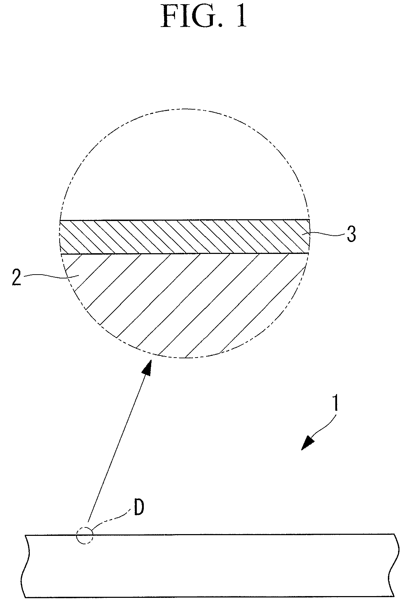

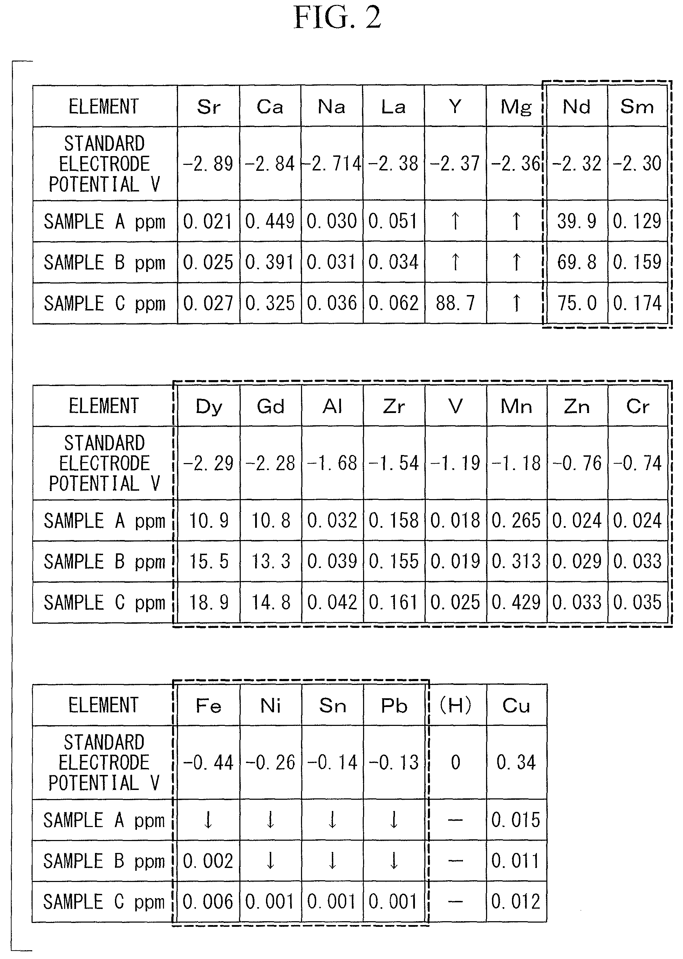

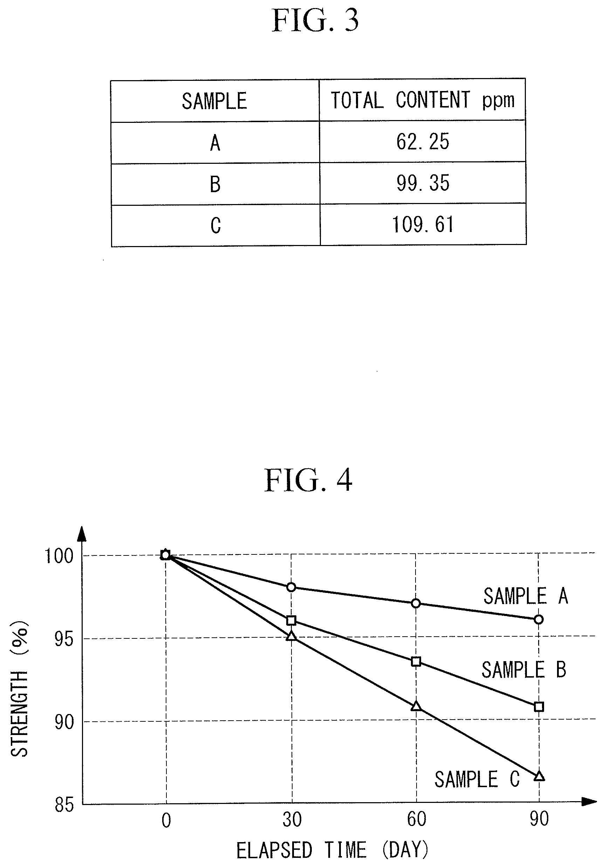

{Fig. 1} Fig. 1 is a partial front view of a biological implant according to an embodiment of the present invention, and an enlarged sectional view showing details of a portion D thereof.
{Fig. 2} Fig. 2 is a diagram showing, in the form of tables, types of metal elements contained in ceramic membranes provided in three samples of the biological implant in Fig. 1, standard electrode potentials thereof, and contents thereof.
{Fig. 3} Fig. 3 is a diagram showing, in the form of a table, total contents of metal elements contained in the ceramic membranes of the samples in Fig. 2, which have a standard electrode potential equal to or greater than -2.35 V and equal to or less than 0 V.
{Fig. 4} Fig. 4 is a diagram showing, in the form of a graph, the relationship between the elapsed time and strength in the case in which the samples in Fig. 2 are immersed in a simulated body fluid.

### {Description of Embodiment}

A biological implant 1 according to an embodiment of the present invention will be described below with reference to the drawings.

As shown in Fig. 1, the biological implant 1 according to this embodiment is provided with a base material 2 made of magnesium or a magnesium alloy and a ceramic membrane 3 formed on a surface of the base material 2.

The ceramic membrane 3 is formed of a material in which the total content of elements having standard electrode potentials equal to or greater than -2.35 V and equal to or less than 0 V is suppressed to a predetermined amount or less. When the total content of the above-described elements is increased, the elements that have been ionized by coming into contact with a body fluid accelerate ionization of magnesium or the magnesium alloy in the base material 2. Specifically, with an increase in the total content of the above-described elements, the base material 2 is more easily corroded, and the mechanical strength thereof is reduced. Therefore, in this embodiment, the total content of the above-described elements is set to be equal to or less than a value at which the base material 2 can keep a desired mechanical strength over a predetermined period after implanting.

Specifically, in this embodiment, an example of the biological implant 1 that is implanted in, as biological tissue, a fractured portion of bone tissue will be described, and the total content is set so that, over the period until the fractured portion is healed, that is, over the period until autogenous bone of the fractured portion is restored, the base material 2 can keep the mechanical strength thereof at 85 % or greater relative to the mechanical strength immediately after implanting.

According to Gurlt, although there is variability among individuals, the period required until the autogenous bone of the fractured portion is restored is about 90 days. In other words, a rapid decrease in the mechanical strength of the biological implant 1 during the period of about 90 days required for the fractured portion to heal is undesirable, and it is desirable to keep the mechanical strength at 85 % or greater.

In the literature "Y. Shikinami, et al. Biomaterials 20 (1999) 859-877", results of a bending strength test performed for the case in which a polylactic-acid osteosynthetic material is immersed in a simulated body fluid (i.e., PBS) are reported, which indicate a 16 % reduction from the initial strength after about 90 days. An initial bending strength of 270 MPa was reported for polylactic acid in the above literature. On the other hand, with the magnesium alloy, the initial bending strength thereof is equal to or greater than 400 MPa, and thus, at a degradation rate that is at least equivalent to that of polylactic acid, that is, with a 15 % or less reduction in the mechanical strength from the initial stage after implanting, by the virtue of having a greater initial bending strength than polylactic acid, it is possible to apply the magnesium alloy to an osteosynthetic treatment that requires an endurance against a load equal to or greater than the treatment range in which existing polylactic-acid osteosynthetic materials are used.

In this embodiment, the ceramic membrane 3 contains oxygen and magnesium as a first main component and a second main component, respectively, and is an anodic oxidation membrane that is generated by an anodic oxidation process performed after molding processing of the base material 2.

With the thus-configured biological implant 1 according to this embodiment, when implanted in, for example, a fractured portion of bone tissue, as an affected area of biological tissue, the ceramic membrane 3 provided at the surface of the base material 2 comes into contact with the body fluid first, thus providing protection so as to suppress corrosion of magnesium or the magnesium alloy forming the base material 2. Because the ceramic membrane 3 also contains minute amounts of metal elements having standard electrode potentials equal to or greater than -2.35 V and equal to or less than 0 V, these metal elements are degraded and ionized in the body fluid.

In this case, with the biological implant 1 according to this embodiment, because the total content of the above-described elements is restricted, the ionized metal elements are restricted so as not to accelerate ionization of magnesium or the magnesium alloy in the base material 2. Consequently, even if the base material 2 is degraded due to corrosion, the rate thereof is restricted to a level such that the mechanical strength is reduced within a range of 15 % or less from immediately after implanting even if 90 days have passed after implanting, and the load exerted on the fractured portion is stably supported by the biological implant 1.

Because the fractured portion is sufficiently restored by the autogenous bone when 90 days have passed after implanting, thereafter, it is permissible that degradation of the magnesium or magnesium-alloy implant advances and that the strength thereof is rapidly reduced. In addition, because the Young's modulus of magnesium or the magnesium alloy is close to the Young's modulus of autogenous bone, even if the metal coexists with autogenous bone over an intermediate to long period, the physical burden that the implant exerts on the autogenous bone would be low. Therefore, because it is unlikely that the implant causes a problem of re-fracture or the like, the degradation slowly advances, and the bone tissue can safely be restored.

In addition, by forming the ceramic membrane 3 by the anodic oxidation process, the ceramic membrane 3 formed of the generated anodic oxidation membrane is firmly bonded with the base material 2 through chemical reactions, thus making it unlikely that the ceramic membrane 3 physically peels off. Consequently, the corrosion resistance thereof is enhanced, and it is possible to prevent the ceramic membrane 3 from peeling after implanting.

Here, ICP atomic emission spectrophotometry in accordance with JIS K 0116 was performed on three samples A, B, and C and the base material 2. With regard to the analysis results for the individual samples A, B, and C, differences from the analysis result for the base material 2 (sample analysis result - base-material analysis result) are shown in Fig. 2 together with detected metal elements and their standard electrode potentials.

The three samples A, B, and C were individually obtained by the anodic oxidation process by using a solution containing ammonium ion and phosphate ion and by using WE43 in accordance with the ASTM standard as the base material 2; in order to control contaminating elements and amounts thereof, pure magnesium was used as the electrode material, and the samples A, B, and C were prepared using the solutions of different purities. Here, WE43 is an Mg-Y-RE-Zr alloy.

In addition, Fig. 3 shows the total contents of metal elements in the individual samples A, B, and C, which have standard electrode potentials equal to or greater than -2.35 V and equal to or less than 0 V. Specifically, these metal elements are at least one type of metal elements selected from Nd, Sm, Dy, Gd, Al, V, Zr, Mn, Zn, Cr, Fe, Ni, Sn, and Pb shown in areas surrounded by broken lines in Fig. 2.

Furthermore, Fig. 4 shows the relationship between the elapsed time and the mechanical strength for the individual samples A, B, and C when immersed in a simulated body fluid (PBS (-) solution at 37.0 °C). With regard to the mechanical strength, the bending strength was measured by performing three-point bending tests in accordance with JIS Z 2248 at elapsed times of 30 days, 60 days, and 90 days after immersing.

The results thereof showed that the total content of the metal elements having standard electrode potentials equal to or greater than -2.35 V and equal to or less than 0 V was the lowest for the sample A at 62.25 ppm, and the reduction in the bending strength after 90 days had passed also remained at about 4 %. The total content for the sample B was 99.35 ppm, and the reduction in the bending strength was about 9 %. Furthermore, the total content for the sample C was the highest at 109.61 ppm, and the reduction in the bending strength was about 13 %.

Specifically, based on these findings, it can be concluded that, by setting the total content of the metal elements having standard electrode potentials equal to or greater than -2.35 V and equal to or less than 0 V to 109 ppm or less, the bending strength after 90 days have passed from the initial stage after implanting can be kept at 85% or greater, that, by setting the total content to 99 ppm, the bending strength after 90 days have passed from the initial stage after implanting can be kept at 90 % or greater, and that, by setting the total content to 109 ppm, the bending strength after 90 days have passed from the initial stage after implanting can be kept at 95 % or greater.

Note that, although WE43 is employed as the base material 2 in this embodiment, alternatively, any alloy classified as AE42, AM60, AS41, AZ31, EZ33, M1, QE22, ZE41, or ZK51 according to the ASTM standard may be employed.

These alloys have relatively low total contents of the metal elements having standard electrode potentials equal to or greater than -2.35 V and equal to or less than 0 V. A feature of these alloys is that, even if membrane-generating processing is applied thereto, as with this embodiment, the amount by which the ceramic membrane is contaminated by the metal elements having standard electrode potentials equal to or greater than -2.35 V and equal to or less than 0 V that are derived from the alloys is suppressed, and thus, it is possible to fabricate a biological implant having a high corrosion resistance.

In addition, although an anodic oxidation membrane generated by means of an anodic oxidation process has been described as an example of the ceramic membrane 3 in this embodiment, alternatively, a membrane generated by chemical conversion coating may be employed.

For example, a crystalline phase of magnesium phosphate may be formed at a surface of the base material 2 by immersing the base material 2 made of pure magnesium in a high-purity (5 mol/L) phosphoric acid solution. In this case, the total content of the metal elements having standard electrode potentials equal to or greater than -2.35 V and equal to or less than 0 V is 39.6 ppm, and the three-point bending strength after 90 days have passed after immersing in PBS is kept at 95.2 % of the value immediately after immersing.

### {Reference Signs List}

- 1: biological implant
- 2: base material
- 3: ceramic membrane

## Claims

1. A biological implant comprising:
a ceramic membrane provided on a surface of a base material made of magnesium or a magnesium alloy,
wherein a total content of a metal element contained in the ceramic membrane, which has a standard electrode potential equal to or greater than -2.35 V and equal to or less than 0 V, is set to be equal to or less than a value at which the base material can keep a desired mechanical strength over a healing period of an implant site in biological tissue.

2. The biological implant according to Claim 1, wherein the desired mechanical strength is 85 % or greater mechanical strength relative to a mechanical strength before implanting.

3. The biological implant according to Claim 2, wherein the total content is equal to or less than 109 ppm.

4. The biological implant according to Claim 3, wherein the total content is equal to or less than 99 ppm.

5. The biological implant according to Claim 4, wherein the total content is equal to or less than 62 ppm.

6. The biological implant according to any one of Claims 1 to 5, wherein the ceramic membrane contains oxygen and magnesium as a first main component and a second main component, respectively.

7. The biological implant according to any one of Claims 1 to 6, wherein the metal element is at least one type of metal element selected from Nd, Sm, Dy, Gd, Al, V, Zr, Mn, Zn, Cr, Fe, Ni, Sn, and Pb.

8. The biological implant according to any one of Claims 1 to 7, wherein the ceramic membrane is generated in a membrane-generating processing step after molding the base material.

9. The biological implant according to Claim 8, wherein the membrane-generating processing step employs a wet process.

10. The biological implant according to Claim 9, wherein the membrane-generating processing step employs an anodic oxidation process.

11. The biological implant according to Claim 9, wherein the membrane-generating processing step employs a chemical conversion coating.
